Europäisches Patentamt

European Patent Office

Office européen des brevets

(11) Publication number: **0 624 798 A1**

# EUROPEAN PATENT APPLICATION

(21) Application number: **94106404.0**

(22) Date of filing: **25.04.94**

(51) Int. Cl.5: **G01N 33/18**

(30) Priority: **28.04.93 US 53374**

(43) Date of publication of application:
**17.11.94 Bulletin 94/46**

(84) Designated Contracting States:
**AT BE DE DK ES FR GB GR IT NL SE**

(71) Applicant: **NALCO CHEMICAL COMPANY**
**One Nalco Center**
**Naperville Illinois 60563-1198 (US)**

(72) Inventor: **Avallone, Stanley C.**
**21665 W. Division Street**
**Lockport, Illinois 60441 (US)**
Inventor: **Meyer, Richard C.**
**6 Dorchester**
**Irnine, California 92720 (US)**

(74) Representative: **Ruschke, Olaf et al**
**Pienzenauerstrasse 2**
**D-81679 München (DE)**

(54) **Monitoring process consumption rates of additives.**

(57) The consumption rate of at least one additive to an industrial process in an industrial process is determined. The additive (22) is added to at least one upstream process stream (12) that feeds into at least one process zone (14) in which the additive is at least partially consumed. Any additive residual leaves the process zone in at least one downstream process stream. The consumption rate is determined by a method comprising:

adding the additive to the process stream or each of the upstream process streams in an additive feed that contains an inert tracer in known proportion to the additive;

monitoring the concentration of the inert tracer in the upstream process stream or each of the upstream process streams (24) and in the downstream process stream (26) or each the downstream process streams;

determining the concentration of the additive residual in the downstream process stream or in each of the downstream process streams;

calculating the rate of additive consumption in the process zone; and

optionally making a consumption-responsive additive feed rate adjustment.

FIGURE 1

## Technical Field of the Invention

The present invention is in the technical field of monitoring the process consumption of additives in industrial processes, such as raw and waste water treatment processes, manufacturing processes and the like, so as to increase the efficiency thereof and provide a consumption-responsive control of additive feed.

## Background of the Invention

The process consumption of additives in industrial processes has been monitored primarily by the determination of the additive residual downstream of the additive-consuming site and then estimating the process demand for the additive from that residual determination together with approximate determinations of additive feed rate and other influences on additive concentration. The rate at which the additive is fed to the industrial process is generally not subject to accurate determination through readings and/or settings on the additive feed system. The additive is at least partly consumed during the process, and the rate of additive consumption is also not subject to accurate determination using the standard industrial process plant controls and/or equipment. The rate of additive consumption is generally determined by a comparison between additive residual levels and the additive feed rate, which again is not precisely known. Operating parameters other than additive consumption in the process may influence the level of additive residual downstream of the additive-consumption site, such as dilution when other additives are charged to the system concentration by evaporation or other means, unaccounted log of fluid from the system and the like. These other influences throw off the consumption rate estimates. This combined lack of accurate additive feed rate readings and lack of accurate additive consumption rates (process demand for the additive) in a industrial process plant severely diminishes the sensitivity of any additive-consumption responsive adjustment of the additive feed rate, and diminishes the ability to follow changes in the additive's consumption rate with appropriate compensations to the additive feed rate. More accurate analysis techniques are generally not practical because feedback information would most probably be days behind the sampling and hence of little value. The process consumption rate of an additive would routinely have changed during the interval.

A sensitive consumption-responsive control of additive feed would render most any industrial process more efficient. Overfeeding of an additive is unnecessarily expensive, may at times diminish the recycling potential of waste water discharged from process and may also at times impair process parameters. Underfeeding of an additive almost inevitably impairs process parameters, and the imbalance between an underfed additive and other materials to a process may squander some of other materials and lower the process output. In some processes an imbalance between the concentrations of materials charged to the process can add to the organic loading of the water discharged from the system and thus be a detrimental factor to the environment and/or a heavier load on a waste water treatment process used to clean up discharged water.

It is an object of the present invention to provide a method or process for monitoring one or more of the process consumption rates of additives to processes that can be conducted on-site in a very short time period. It is an object of the present invention to provide a method or process for monitoring at least one process consumption rate of an additive to an industrial process together with the additive's feed rate and thereby provide a sensitive consumption-responsive control of such feed rate that can be conducted on-site in a very short time period. It is an object of the present invention to provide an industrial process wherein one or more of the process consumption rates of additives is monitored on-site in a very short time period. It is an object of the present invention to provide an industrial process wherein at least one process consumption rate of an additive to the process, together with the additive's feed rate, is determined and a sensitive consumption-responsive control of such feed rate is provided on-site in a very short time period. These and other objects of the present invention are discussed in detail below.

## Disclosure of the Invention

The present invention provides a process for the monitoring of at least one additive consumption rate of an industrial treatment process comprising adding an inert tracer, for instance a fluorescent specie(s), to the additive feed in known proportion to the additive, monitoring the level of such inert tracer both ahead (upstream) of and behind (downstream of) the additive-consumption site, determining the level of additive residual downstream of the additive-consumption site, and based on such determinations calculating the rate of additive consumption and optionally calculating a consumption-responsive additive feed rate adjustment, and an industrial treatment process that includes such monitoring and computation steps.

Brief Description of the Drawings:

FIGURE 1 is a schematic depiction of a waste water treatment plant employing the process of the present invention.

FIGURE 2 is a schematic depiction of a boiler water treatment plant employing the process of the present invention.

FIGURE 3 is a schematic depiction of a manufacturing process plant employing the process of the present invention.

FIGURE 4 is a schematic depiction of a manufacturing process plant employing the process of the present invention.

FIGURE 5 is a schematic depiction of a manufacturing process plant employing the process of the present invention.

Preferred Embodiments of the Invention

The present invention includes the monitoring of the inert tracer(s) in the additive stream of the industrial treatment process. By the term "monitoring" is meant herein, unless expressly indicated otherwise, the determination of the concentration of the inert tracer(s) in a sample or a value that is proportionate to such concentration. Such monitoring can be conducted on a singular, intermittent, semi-continuous or continuous basis, and preferably is conducted on-site (at the site of the industrial process plant).

The present invention comprises the monitoring of at least one additive consumption rate of the industrial treatment process by employing the information provided by the monitoring of the inert tracer(s). The additive consumption rate monitored by the process of the present invention may be any additive consumption rate within the industrial process, and its determination by the process of the present invention is not effected by influences on the additive stream, such as mixtures of multiple streams, carry over, cross contamination, leaks between systems, flow path variations, other dilutions, concentrations and the like.

By "monitoring an additive consumption rate" is meant herein, unless expressly indicated otherwise, a determination of a value of an additive's consumption rate, which value may be a relative value, a substantially quantitative value, or an approximate quantitative value. The monitoring of the process of the present invention combines at least some of the information provided by the monitoring of the inert tracer(s) ahead of and behind the process with at least some of the information available concerning the addition of the inert tracer(s) to the additive feed and the additive residual level determination.

The inert tracer(s) concentration at the sampling sites depends on the effects on the inert tracer(s) between its introduction (together with the additive) and sampling sites. Such information discloses the feed rate of the additive and the zero-consumption level of additive at the downstream sampling site, and from such zero-consumption additive level and the actual additive residual level the additive's consumption rate can be computed. The process of the present invention thus also permits an accurate determination of the degree of any consumption rate changes and feed rate compensations. This information not only permits a more accurate and efficient responsive feed rate adjustment, but also provides an alert for upsets in the process which will trigger an abnormal change in the additive's consumption rate. The information provided by the monitoring of the additive's consumption rate is of great importance all process operating parameters related to such consumption rate.

Some processes may have a plurality of process consumption sites for a given additive, and the separate consumption rates at each consumption site can be provided by the use of the process of the present invention, preferably by the monitoring of the inert tracer(s) at a plurality of sites, one upstream of and one downstream of each consumption site or potential consumption site, together with the additive residual determinations needed for the consumption rate computations.

For instance, the known proportion between the inert tracer concentration in the additive feed ("$C_T^I$") and the active additive concentration in the additive feed ("$C_A^I$") can be expressed as in Formula 1:

Formula 1 $\qquad C_A^I = k\ C_T^I$

wherein k is a constant. The rate of active additive feed can be determined from the concentration of the inert tracer at the upstream sampling site ("$C_T^U$") using Formula 2:

Formula 2 $\qquad C_A^U = k\ C_T^U$

wherein $C_A^U$ is the concentration of the active additive at the upstream sampling site, and thus a value equivalent to the additive feed rate, and k is a constant of the same value as in Formula 1. At the sampling site downstream of the process the post-process or downstream inert tracer concentration ("$C_T^D$") and the downstream residual concentration of the additive ("$C_A^D$") are both determined. The process consumption rate of the additive can computed using Formula 3:

Formula 3 $\qquad (k\ C_T^D - C_A^D)\ /\ C_A^U$ = additive con-

sumption rate

wherein k is a constant having the same value as in Formulas 1 and 2 above, and $k \, C_T^D$ is the hypothetical zero-consumption concentration of the additive downstream. In many instances $C_T^D$ will be substantially the same as $C_T^U$.

The above formulas are not tied to time units. By feed rate is meant the concentration of the additive in the process stream, rather than amount added per time unit. Likewise by consumption rate is mean the proportion of the amount fed that is consumed.

Some processes may have a plurality of additives for which the computation of accurate consumption rates would improve process efficiency. Such process consumption rates of the plurality of additives may be evaluated by using a plurality of inert tracers, each of which are introduced with different additives in known proportion thereto, and the additive may be fed to the process at the same point or at sites along separate streams or even after the additives are combined.

Some processes may have a plurality of process zones in each of which an additive is partially consumed, which process zones may be disposed sequentially or in parallel, or a plurality of process streams that each feed a portion of the additive to a process zone, or a plurality of process streams that carry a portion of the additive residual away from the process zone. The present invention can be employed in any of these situations or combinations of situations, provided that the concentration of the inert tracer is monitored across the process zone for which a separate consumption rate is to be determined and the additive residual is determined downstream of the process zone for which a separate consumption rate is to be determined without any intervening additive-consumption zones.

The industrial processes for which the present invention may be used are processes of any industry which employ at least one additive subject to consumption in a water-based process, including without limitation a process that purifies a raw water stream and/or system for makeup use, a process that treats waste materials and/or waste waters, a process that separates solids from liquids, a manufacturing process, particularly a chemical industry manufacturing process, including without limitation the pulping and papermaking industries, the steel industry, the metal working industries, the food processing industries, the mining industries, the automotive industry, the textile industry, utilities, chemical processing industries, manufacturing industries, spray paint industries, refining industries such as the refining of aluminate, and the like.

The solids and/or solutes within the waters of these processes may be substantially or mainly organic, or substantially or mainly inorganic, or a mixture of both organic and inorganic materials. The process of the present invention would generally not be applicable to an industrial process wherein the process water stream and/or system has a high solids loading, for instance a solids loading in excess of 40%.

A process water stream or system may contain dissolved solids or dissolved gases, or it may be a slurry (dilute or concentrated), or it may be a slurry containing dissolved solids and/or gases. A process water stream and/or system may also contain liquids other than water, which liquids may be miscible or immiscible with water.

A raw or waste water treatment process, as that terminology is used herein and as that terminology is understood in the raw and waste water treatment field, is a process in which a raw or waste water stream and/or system is treated to separate the water stream and/or system into a plurality of fractions, one of which fractions having a higher concentration of at least one of the initial components. The separation technique(s) most commonly employed are one or more of solute/solid conversions, liquid/liquid separations, gas/liquid separations, solid/liquid separations, although gas/gas separations and solid/solid separations and other more refined techniques are at times encountered. When a raw or waste water stream and/or system contains solids, the initial waste water treatment techniques employed are very often that of bulk solid/liquid separation, for instance by filtration (gravity or vacuum assisted, air flotation-assisted, screening, straining belt-pressing and the like), settling, clarification or centrifugation, or combinations thereof, and such bulk separation techniques may be enhanced by the use of coagulants, flocculants and other solids-concentrating active chemical additives. The water phase may be concentrated or treated (for instance by a pH adjustment with an acid or base additive) to decrease the dissolved solids content thereof or release emulsified components by means of emulsion breaking, before or after such bulk solids separation techniques are applied. The solids may be further dewatered using an additive to effectuate or enhance the dewatering. Dissolved or entrained gases may be separated from the liquid, liquids may be fractionated, solids may be fractionated, and so forth, commonly with the addition of a process-enhancing additive. The primary feature of raw and waste water treatment processes is that a water stream and/or system having a plurality of components (including the water thereof) is separated into a plurality of fractions, and at least one of those fractions has a higher concentration of at least one of such components than the original water stream and/or system. When the technique employed is one of the solid/liquid separation techniques, the resultant fractions typically include one having a high concentration of solids, and one having a low concentration of solids and thus a higher concentration of water.

The ultimate fractions produced in a typical waste water treatment process are destined either for recycle to the process generating the waste or to a different process, or for disposal. These "products" of a waste water treatment plant seldom have a value commensurate with that of the process generating the waste. A typical waste water treatment plant is therefore extremely sensitive to the economics of the treatment process involved, ad unlike the average process plant, its influent is extremely variable and its additive feed and monitoring means are far less sensitive than that of a process plant. The process of the present invention, enables a plant to be proactive in its additive feed adjustments and additive consumption monitorings, and abnormal additive consumption determinations will alert plant operators of inlet water quality upsets.

Raw water streams and/or systems for the purposes of the present invention are waters being prepared for addition to, and use in, a process or cooling water system or boiler stream, and include without limitation well water, river water and other surface water supplies. Waste waters and/or systems for purposes of the present invention are most often waters that have been discharged from a prior process or cooling water system or boiler stream. (Some sewer waters are waters that have been discharged from a prior process, while other sewer waters are not and yet are within the category of waste waters for the purposes of the present invention.)

Both raw and waste water treatment processes are clarification or waste reduction processes by which the water is to some degree purified. The present process is of course not limited to water purification processes. For instance, the present invention may be applied to monitor the consumption rate of sodium hydroxide in a papermill Kraft cycle. The present invention may be applied to monitor the consumption rate of a wet end additive in a papermaking process. The present invention may be applied to monitor the consumption rate of an internal sizing in the textile industry. The present invention may be applied to monitor the consumption rate of a downhole corrosion inhibitor in the oil and gas well industry. The present invention may be applied to monitor the consumption rate of additives employed in ore beneficiation processes. The present invention may be applied to monitor the consumption rate of additives employed in chemical refining processes.

The process water streams and/or systems of typical process can be characterized by the following physical and/or chemical property ranges, although the present invention is not limited to processes within such ranges:

a pH of from about 2 to about 12;

a temperature of from about 5°C (41 °F) to about 245 °C (500 °F);

an insoluble solids content of from about 1 ppm to about 1,000 ppm; and

a total solids content of from about 100 ppm to about 100,000 ppm.

## Inert Tracers

The inert tracer must be both soluble and stable in the additive feed and transportable with the water of the process system and thus wholly water-soluble therein at the concentration it is used, under the temperature and pressure conditions to be encountered. Preferably the selected inert tracer also meets the following criteria:

1. Be thermally stable and not decompose at the temperature within the given process;

2. Be detectable on a continuous or semicontinuous basis and susceptible to concentration measurements that are accurate, repeatable and capable of being performed on process water;

3. Be substantially foreign to the chemical species that are normally present in the water of the process systems in which the inert tracer may be used;

4. Be substantially impervious to interference from, or biasing by, the chemical species that are normally present in the water of the process systems in which the inert tracer may be used;

5. Be substantially impervious to any of its own potential specific losses from the water of the process system, including selective carry-over;

6. Be compatible with all treatment agents employed in the water of the process systems in which the inert tracer may be used, and thus in no way reduce the efficacy thereof;

7. Be compatible with all components of the additive feed formulation despite the concentrations of the tracer and/or other components in such a formulation, and despite any storage and/or transportation conditions encountered; and

8. Be reasonably nontoxic and environmentally safe, not only within the environs of the water of the process system in which it may be used, but also upon discharge therefrom.

In preferred embodiment, the chemical compound(s) selected as an inert tracer(s) should not be one that is consumed or selectively lost to the water of the process system, for instance due to degradation, deposition, complexation, or other phenomena, unless such loss is at a rate that is predictable and proportional to a non-process-consumption loss of the additive being monitored. The inert tracer(s) used in the present invention is preferably substantially unconsumed in the process environment. An inert tracer(s) that is wholly inert in the process environment would not react with any of the components in the water of the process system to which it is added, would not degrade in the environment of the water of the process system, would be incapable of coupling and/or depositing in any manner within such process system and would not appreciably be effected by other system parameters such as metallurgical composition, heat changes or heat content. There are water-soluble inert tracer(s) that are wholly inert, or substantially inert, in the aqueous environments likely to be encountered in industrial process systems. Further, it is believed that an inert tracer(s) having a degree of inertness such that no more than 10 weight percent thereof is lost due to reaction, degradation, coupling and/or deposition during the time that elapses between its addition and its final monitoring as a process stream component, is sufficiently, or substantially, inert for the purpose of the present invention for most, if not all, additive consumption monitorings.

Generally it is desirable to employ the least amount of inert tracer that is practical for the circumstance, and the amount of the inert tracer added to the additive feed should be at least a amount effective for the determinations desired. Seldom would a inert tracer be deliberately used in a amount grossly in excess of the minimum effective amount because there generally would be no practical purpose in doing so that would justify the costs involved and any deleterious effects on the quality of the water of the process or on the process itself caused by the presence of the inert tracer therein. The amount of inert tracer to be added to the additive feed that is effective without being grossly excessive will vary with a variety of factors including, without limitation, the inert tracer and monitoring method selected, the potential for background interference with the selected monitoring method, the magnitude of the expected inert tracer concentration at the downstream sampling/monitoring station, the monitoring mode (which preferably would be an on-line substantially continuous monitoring mode), and other similar factors. Generally the dosage of an inert tracer to a additive feed will be at least sufficient to provide a concentration of tracer at the downstream sampling/monitoring station of at least about 0.5 ppb, and more commonly at least about 5 ppb or higher, up to about 100 or 200 ppm, in the process stream.

The additive feed is commonly, but not always, comprised of the active additive and one or more inert diluents. A diluent is frequently a solvent for the additive, and such solvent can, and in may instances is, water. A diluent, particularly a solvent, is frequently included in the additive feed to facilitate the rapid and substantially homogeneous distribution of the active additive in the process stream to which the additive feed is charge. In preferred embodiment the active additive is a commodity process additive such as ammonia, caustic, phosphate, ferric chloride, alum and sulfuric acid. These naturally occurring or simply produced commodity additives are employed in bulk amounts, for instance at concentrations within the rage of from about 0.01 to about 10 weight percent in a process mixture. The weight ratio of active additive to inert tracer in the additive feed thus is often within the range of from about 10:1 to about 100:1. The weight ratio between the active additive ad the inert tracer in a process stream ahead of the additive-consuming process is of course substantially the same as that of the additive feed, and thereafter that weight ratio would fall as the additive is consumed in the process, for instance to the extent of approaching a 1:1 weight ratio or less.

The concentration of the active additive in the additive feed is generally from about 5 to about 100 weight percent. If the concentration of the active additive in the normal feed formulation is inconsistent with inert tracer stability for the normal feed holding time, the holding time could be shortened or the feed formulation could be diluted to diminish the potential stability concerns. If the additive is charged to a additive feed as a component of an additive concentrate, the same stability considerations noted herein for the additive feed would generally apply unless the concentrate was in a substantially dry form.

As noted above, the inert tracer must be added to the additive feed in known proportion to the additive, and preferably the inert tracer is in into the process system together with the additive feed at a known and constant concentration therein which is at a known and constant proportion to the additive actives therein. The preferred method of achieving such proportionality is to formulate the inert tracer together with additive concentrate if the additive feed is to be prepared by on-site dilution and if the inert tracer is stable in such concentrate. The concentrate may be a aqueous solution or other substantially homogeneous admixture that disperses with reasonable rapidity in the dilution fluid which is added. Since in most any instance an additive and the inert tracer would both be added to a process system as components of a fluid feed formulation, rather than as a dry solid or individual neat liquids, the tracer concentration may be correlated not to the numerical concentration of the inert tracer itself or the additive itself, but instead to the concentration of a formulated product containing the additive, which in turn can be correlated to the concentration of the inert tracer and/or additive when and if such information is required. Therefore the proportionality of the tracer to the additive feed for the purposes of the present invention can be equivalent to a proportionality of tracer to the active additive component of the feed. The correlation between inert tracer concentration and additive concentration is of course a correlation based on zero-consumption of additive regardless of whether such zero-consumption rate is actual (at the sampling station ahead of the process) or is a hypothetical standard (at the sampling station downstream of the process).

Among these substantially process-system inert fluorescent compounds are the mono-, di- and trisulfonated naphthalenes, including their water-soluble salts, particularly the various naphthalene mono- and disulfonic acid isomers, which are preferred inert tracers for use in the present invention. The naphthalene mono- and disulfonic acid isomers are water-soluble, generally available commercially and easily detectable ad quantifiable by known fluorescence analysis techniques. Preferred naphthalene mono- and disulfonic acid isomers are the water-soluble salts of naphthalene sulfonic acid ("NSA"), such as 2-NSA, and naphthalene disulfonic acid ("NDSA" or "NDA"), for instance 1,5-NDSA. Many of these inert tracer(s) (mono-, di- and trisulfonated naphthalenes and mixtures thereof) are extremely compatible with the environments of most process systems. Among these preferred fluorescent tracers, 2-NSA and 1,5-NDSA have been found to be thermally stable (substantially inert) at temperatures up to at least about 540 °C (1004 °F), for at least 24 hours at 285 °C (545 °F) and at pressures up to about 1,500 psig for time periods at least commensurate with, and often well in excess of, commercial process holding times. Such inert fluorescent tracers are not selectively carried over into steam.

Another group of inert fluorescent tracers that are preferred for use in the process of the present invention are the various sulfonated derivatives of pyrene, such as 1,3,6,8-pyrene tetrasulfonic acid, and the various water-soluble salts of such sulfonated pyrene derivatives.

In preferred embodiment the inert tracer is one of these sulfonated fluorescent tracers and are employed at concentration levels of from about 0.5 ppb, and more commonly at least about 5 ppb or higher, up to about 5 or 7 ppm in the process stream.

By the terms "tracing" is meant herein, unless expressly indicated otherwise, the determination of the concentration of the inert tracer(s) in the process stream. Such tracing would seldom be conducted on a singular, intermittent or semi-continuous basis for the purpose of the present invention, but instead on a substantially continuous basis, and preferably the concentration determination is conducted on-site (at the site of the process system) to provide rapid determinations of additive feed rate and consumption rate. The inert tracer is at times referred to herein merely as a "tracer". The term "process stream" as used herein, unless expressly indicated otherwise, refers to a stream feeding into a process, a stream within a process and a stream discharged from a process, each of which are a process stream at a point along the normal process stream route.

The tracer is preferably selected from among those that are easily quantifiable by a fluorescence analysis method, a preferred analytical technique for the purposes of the present process. Other analysis methods not excluded for use in quantifying the inert tracer are HPLC and fluorescence analysis combinations, which are described in more detail below.

Fluorescence Emission Spectroscopy

The detection and quantification of specific substances by fluorescence emission spectroscopy is founded upon the proportionality between the amount of emitted light and the amount of a fluoresced substance present. When energy in the form of light, including ultra violet and visible light, is directed into a sample cell, fluorescent substances therein will absorb the energy and then emit that energy as light having a longer wavelength than the absorbed light. A fluorescing molecule absorbs a photon resulting in the promotion of an electron from the ground energy state to an excited state. When the electron's excited state relaxes from a higher energy vibrationally-excited state to the lowest energy vibrationally-excited state, energy is lost in the form of heat. When the electron relaxes to the ground electronic state, light is emitted at a lower energy than that absorbed due to the heat-energy loss, and hence at a longer wavelength than the absorption. The amount of emitted light is determined by a photodetector. In practice, the light is directed into the sample cell through an optical light filter so that the light transmitted is of a known wavelength, which is referred to a the excitation wavelength and generally reported in nanometers ("nm"). The emitted light is similarly screened through a filter so that the amount of emitted light is measured at a known wavelength or a spectrum of wavelengths, which is referred to as the emission wavelength and generally also reported in nanometers. When the measurement of specific substances or categories of substances at low concentrations is desired or required, such as often is the case for the process of the present invention, the filters are set for a specific combination of excitation and emission wavelengths, selected for substantially optimum low-level measurements.

In general, the concentration of an inert tracer can be determined from a comparison of a sample's emissions intensity to a calibration curve of the given tracer's concentration versus emissions, for the same set of excitation wavelength/emission wavelengths. Such a concentration-by-comparison method by which the sensed emissions are converted to a concentration equivalent preferably is employed to determine concentrations of an inert tracer that are within the concentration range over which a linear emission response is observed, and this concentration range is referred to herein as the "linear-emission-response concentration range". The linear-emission-response concentration range is to some extent dependent upon the specific inert tracer and the excitation wavelength/emission wavelength set employed. At inert tracer concentrations higher than a given inert tracer's linear-emission-response concentration range, there is a negative deviation from ideal (linear) behavior, the degree of emission for a given concentration being less than predicted by a linear extrapolation. In such instances, the sample can be diluted by known factors until the concentration of the inert tracer therein falls within the linear-emission-response concentration range. If the inert tracer is present in the sample at only very low concentrations, there are techniques for concentrating the inert tracer by known factors until its concentration falls within the linear-emission-response concentration range or is otherwise more readily measured, for instance by liquid-liquid extraction. Nonetheless, preferably a calibration curve over the linear-emission-response concentration range would be prepared or obtained before employing a given inert tracer, and preferably the inert tracer would be added to the additive feed in an amount sufficient to provide a concentration of the inert tracer in the blowdown that is within the linear-emission-response concentration range. Generally the linear-emission-response concentration range of an inert tracer is sufficiently broad to readily estimate the concentration of the inert tracer in the additive feed that will be sufficient for this purpose. A linear-emission-response concentration range will most often extend through a concentration range from a concentration of "m" to a concentration of at least 10m.

A determination of the concentration of a fluorescent inert tracer in a process stream can be made when the concentration of the inert tracer in the process steam is only several parts per million (ppm) or even parts per billion (ppb) for some of the inert tracers that can be employed in the process of the present invention. In preferred embodiment, the amount of a fluorescent inert tracer added to the additive feed should be sufficient to provide a concentration of the inert tracer in the process steam downstream of the process (the last process steam to be analyzed) of from about 5 ppb to about 5 or 7 ppm. Such analyses (the measurements of the light emitted in response to the light transmitted to the process steam) can be made on-site, preferably on an almost instant and continuous basis, with simple portable equipment.

As mentioned above, at times it may be desired to employ a plurality of inert tracers. For instance, it may be desired to monitor more than one process additive, and a separate inert tracer may be used for each monitored additive, and thus the separate consumption rates of either, and preferably both, would be determined by the process of the present invention. In other instances it may be desired to use a plurality of inert tracers solely for the monitoring of a single additive, for instance to confirm that neither tracer is undergoing any tracer-specific loss. Such separate and distinct inert tracers can each be detected and quantified in a single water process stream sample despite both being fluorescent tracers if their respective wavelengths of emission do not interfere with one another. Thus concurrent analyses for multiple inert tracers is possible by selection of inert tracers having appropriate spectral characteristics. Preferably widely separated wavelengths of radiation should be used to excite each of the inert tracers, and their fluorescent emissions should be observed and measured at widely separated emission wavelengths. A separate concentration calibration curve may be prepared or obtained for each inert tracer. In other words, more than one inert tracer can be employed, and then the presence and/or concentration of each such inert tracer in the process stream may be determined using analytical parameters (particularly the excitation/emission wavelengths) effective for each such inert tracer, which analytical parameters preferably are sufficiently distinct to differentiate between measurements. Since a plurality of inert tracers may be separately but concomitantly monitored, the present invention does not exclude the use of one or more additional inert tracers for purposes other than the present invention, nor does it exclude the concomitant use of an inert tracer for purposes of the present invention and for some other purpose.

Fluorescence emission spectroscopy on a substantially continuous basis, at least over a given time period, is one of the preferred analytical techniques for the process of the present invention. It is one of the preferred analysis techniques for quantifying and determining the concentration of the inert tracer in a process stream for monitoring process additives and it is an analysis technique having significant advantages. Fluorescent chemical tracers and monitoring techniques are now known, for instance as disclosed in U.S. Patent No. 4,783,314, J. E. Hoots and B. E. Hunt, issued November 8, 1988, incorporated herein by reference, wherein inert fluorescent tracers are employed in combination with a fluorescence monitoring, such as the sodium salt of 2-naphthalenesulfonic acid.

When the inert tracer is 2-NSA, one of the water-soluble salts of naphthalene sulfonic acid ("NSA"), its concentration in the process stream from a process stream can be fluorometrically measured by excitation at 277 nm and emission measurement at 334 nm, and the emissions observed referenced to a standard aqueous solution containing 0.5 ppm 2-NSA, as acid actives.

A Gilford Fluoro IV dual-monochromator spectrofluorometer can be used for a fluorometric analysis conducted on an intermittent basis and for on-line fluorescence monitoring, a portable fluorometer equipped with appropriate excitation and emission filters and a quartz flow through cell can be used, such as is commercially available from Turner Designs (Sunnyvale, California) Model Fluorometer 10AU, which has a flow-pressure rating of 72 psi, an 0.3-centimeter diameter and a two-inch long flow cell.

In general for most fluorescence emission spectroscopy methods having a reasonable degree of practicality, it is preferable to perform the analysis without isolating in any manner the inert tracer. Thus there may be some degree of background fluorescence in the process stream on which the fluorescence analysis is conducted, which background fluorescence may come from chemical compounds in the process stream that are unrelated to the present process. In instances where the background fluorescence is low, the relative intensities (measured against a standard fluorescent compound at a standard concentration and assigned a relative intensity for instance 100) of the fluorescence of the tracer versus the background can be very high, for instance a ratio of 100/10 or 500/10 when certain combinations of excitation and emission wavelengths are employed even at low fluorescent compound concentrations, and such ratios would be representative of relative performance (under like conditions) of respectively 10 and 50. In preferred embodiment, the excitation/emission wavelengths and/or the amount of tracer employed are selected to provide a relative fluorescence of at least about 5 or 10 for the given background fluorescence anticipated.

For instance, for most process stream backgrounds, a compound that has a relative performance of at least about 5 at a reasonable concentration is very suitable as an inert tracer. When there is or may be a specific chemical specie of reasonably high fluorescence in the background, the tracer and/or the excitation and/or emission wavelengths often can be selected to nullify or at least minimize any interference of the tracer measurement(s) caused by the presence of such specie.

One method for the continuous on-stream monitoring of chemical tracers such as the inert tracer by fluorescence emission spectroscopy and other analysis methods is described in U.S. Patent No. 4,992,380, B. E. Moriarity, J. J. Hickey, W. H. Hoy, J. E. Hoots and D. A. Johnson, issued February 12, 1991, incorporated hereinto by reference.

Combined HPLC-Fluorescence Analysis

The combination of high-pressure liquid chromatography ("HPLC") and fluorescence analyses of fluorescent tracers is a powerful tool for the present process, particularly when very low levels of the inert tracer are used or the background fluorescence encountered would otherwise interfere with the efficacy of the fluorescence analysis. The HPLC-fluorescence analysis method allows the tracer compound to be separated from the fluid matrix and then the tracer concentration can be measure. The combination of HPLC-fluorescence analysis is particularly effective for measuring minute levels of tracer in highly contaminated fluids.

Analytical techniques for quantifying the presence and/or concentration of a chemical specie without isolation thereof are within an evolving technology, and the above survey of reasonable analytical techniques for use in monitoring the inert tracer in the process of the present invention may presently not even be exhaustive, and most likely techniques equivalent to the above for the purposes of the present invention will be developed in the future.

As noted above, in preferred embodiment, the chemical compound(s) selected as the inert tracer should be soluble in the process stream to which it is added and should be either stable in the environment thereof for the useful life expected of the inert tracer, or its loss from the process stream due to degradation, deposition, complexation, or other phenomena should be predictable and compensative, particularly since it is desired not merely to detect the presence of some amount of the inert tracer, but also to determine the concentration thereof that is equivalent to a zero-consumption rate. In preferred embodiment, the combination of the chemical compound(s) selected as the inert tracer and the analytical technique selected for determining the presence of such tracer, should permit such determination without isolation of the inert tracer, and more preferably should permit such determination on a continuous and/or on-line basis.

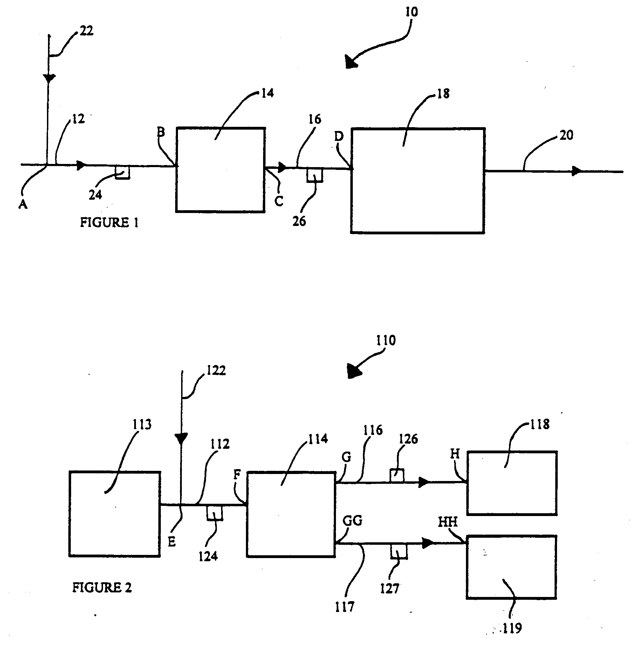

Referring now to FIGURE 1, there is shown schematically a waste water treatment plant 10 including a waste stream line 12 which feeds into a biological degradation process 14 at point B, a transport line 16 from the biological degradation process 14 (commencing at point C) which feeds into a clarifier 18 at point D, and a discharge line 20 from the clarifier 18. Along the waste stream line 12 is an ammonia feed line 22 , which introduces ammonia to the waste stream in the waste stream line 12 at point A ahead of the biological degradation process 14. Ammonia is consumed during the processing of the waste in the biological degradation process 14. The ammonia that is introduced to the waste stream at point A contains an inert tracer in known proportion to the ammonia in the ammonia feed. Between points A and B is a first sampling/monitoring station 24 which preferably, as shown, is on-line. Between points C and D is a second sampling/monitoring station 26 which preferably, as shown, is also on-line. This waste water treatment plant 10 has the capability to monitor the concentration of the inert tracer at such first and second sampling/monitoring stations 24, 26, and has the capability to measure the level of residual ammonia at the second sampling/monitoring station 26 by conventional techniques. The ammonia feed rate and rate of ammonia consumption can be measured. The first sampling/monitoring station 24 determines the ammonia feed rate based on the concentration of the inert tracer in the process stream at that point and the known proportion between the level of such inert tracer and the level of ammonia as this combination is fed to the waste stream line 12. The second sampling/monitoring station 26 again measures the concentration of the inert tracer and, by the known proportion between the level of such inert tracer and the level of ammonia as this combination is fed to the waste stream line 12, provides a determination of what the level of the ammonia would be at this second sampling/monitoring station 26 if no ammonia had been consumed in the biological degradation process 14. The difference between the actual residual ammonia level as measured at the second sampling/monitoring station 26 and such zero-consumption ammonia level determination provides the actual ammonia consumption rate. The actual ammonia consumption rate is determined free of other changes in the concentration of ammonia ahead of the second sampling/monitoring station 26, such as dilution when other additives are charged to the biological degradation process 14 in separate feed lines, or into the waste stream line 12 downstream of point B, concentration by evaporation or other means, unaccounted loss of fluid from the biological degradation process 14 and the like. The inert tracer is subjected to these other influences in a degree in proportion to that of the ammonia, while the ammonia alone is consumed by the process within the biological degradation process 14, leaving the inert tracer level as a standard by which the ammonia consumption can be measured.

Referring now to FIGURE 2, there is shown schematically a boiler water treatment plant 110 including a demineralized water line 112, leading from a demineralizer system 113, which feeds into a deaerator 114 at point F, a first and second transport line 116, 117 from the deaerator 114 (commencing at points G and GG) each of which feeds into a first and second boiler 118, 119 at points H and HH respectively. Along the demineralized water line 112 is an sodium hydroxide feed line 122 , which introduces sodium hydroxide to the demineralized water line 112 at point E ahead of the deaerator 114. Sodium hydroxide is consumed during the processing of the water in the deaerator 114. To provide a sensitive sodium hydroxide-consumption determination and to follow changes in the sodium hydroxide consumption with appropriate changes in the sodium hydroxide feed rate, the sodium hydroxide that is introduced to the demineralized water at point E contains an inert tracer in known proportion to the sodium hydroxide in the sodium hydroxide feed. Between points E and F is a first sampling/monitoring station 124 which preferably, as shown, is on-line. Between points G and H is a second sampling/monitoring station 126. Between points GG and HH is a third sampling/monitoring station 127. In this boiler water treatment plant 110, having the capability to monitor the concentration of the inert tracer at such first, second and third sampling/monitoring stations 124, 126, 127, and having the capability to measure the level of sodium hydroxide at the second and third sampling/monitoring station 126, 127, the sodium hydroxide feed rate and rate of sodium hydroxide consumption can be measured. The first sampling/monitoring station determines the sodium hydroxide feed rate based on the concentrations of the inert tracer at that point and the known proportion between the level of such inert tracer and the level of sodium hydroxide as this combination is fed to the demineralized water line 112. The second and third sampling/monitoring stations 126, 127 both measure the concentration of the inert tracer and by the known proportion between the level of such inert tracer and the level of sodium hydroxide, as this combination is fed to the demineralized water line 112, provide a determination of what the level of the sodium hydroxide would be at these second and third sampling/monitoring stations 126, 127 if no sodium hydroxide had been consumed in the deareator 114. The difference between the actual combined residual sodium hydroxide levels as measured at the second and third sampling/monitoring stations 126, 127 and combined zero-consumption sodium hydroxide level determinations provide the actual sodium hydroxide consumption rate. The sodium hydroxide feed rate can be adjusted to better conform to actual consumption rate and/or to compensate for changes detected in the consumption rate, and the accuracy of such feed rate adjustment can be determined at the first sampling/monitoring station 124.

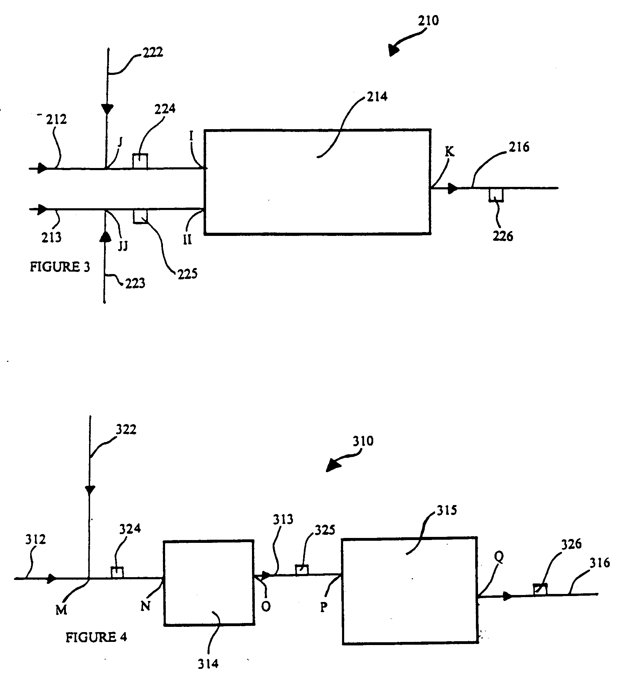

Referring now to FIGURE 3, there is shown schematically a process plant 210 including a first and second raw material line 212, 213 which both feed into a process 214 at points I and II respectively, a product outlet line 216 from the process 214 (commencing at point K) which discharges the product. Along the first and second raw material line 212, 213 are ferric chloride feed lines 222, 223 respectively, each of which introduces ferric chloride to the raw material at points J and JJ respectively ahead of the process 214. The ferric chloride is consumed during the processing of the raw material in the process 214. The ferric chloride that is introduced to the raw material at points I and II respectively contains an inert tracer in known proportion to the ferric chloride in the ferric chloride feed Between points I and J is a first on-line sampling/monitoring station 224. Between points II and JJ is a second on-line sampling/monitoring station 225. Between points K and L is a third on-line sampling/monitoring station 226. The concentration of the inert tracer is monitored at such first, second and third sampling/monitoring stations 224, 225, 226 and the level of residual ferric chloride is determined by conventional techniques at the third sampling/monitoring station 226. The first and second sampling/monitoring stations 224, 225, in combination determine the total ferric chloride feed rate based on the concentrations of the inert tracer at those points. The third sampling/monitoring station 226, which again measures the inert tracer concentration, by the known proportion between the inert tracer and the ferric chloride as fed to the first and second raw material lines 212, 213, provides a determination of the hypothetical zero-consumption level of the ferric chloride at this third sampling/monitoring station 226. The difference between the actual residual ferric chloride level, which is also measured at the third sampling/monitoring station 226, and zero-consumption ferric chloride level determination provides the ferric chloride consumption rate.

Referring now to FIGURE 4, there is shown schematically a process plant 310 including a raw material line 312 which feeds into a first process 314 at point N, an intermediate transport line 313 which receives a intermediate process stream from the first process 314 at point O and feeds into a second process 315 at point P, a product outlet line 316 from the second process 315 (commencing at point Q) which discharges the product. Along the raw material line 312 is an amine feed line 322, which introduces the amine feed to the raw material stream at point M ahead of the first process 314. The amine is partly consumed during the processing of the raw material in the first process 314 and partly consumed during the processing of the intermediate in the second process 315. The amine feed that is introduced to the raw material at point M contains a inert tracer in known proportion to the amine. Between points M and N is an on-line first sampling/monitoring station 324. Between points N and O is an on-line second sampling/monitoring station 325. Downstream of point Q is a third sampling/monitoring station 326. In this process plant 310 the concentration of the inert tracer is being monitored at each of the first, second and third sampling/monitoring stations 324, 325, 326, and the amine feed rate and separate rates of amine consumption in the first and second processes 314, 315 are being measured. The first sampling/monitoring station 324 determines the amine feed rate to the raw material stream. The second sampling/monitoring station 325 monitors the concentration of the inert tracer providing the zero-consumption amine level determination and the actual amine residual concentration, providing the amine consumption rate. The inert tracer concentration and the amine residual concentration is also measured at the third sampling/monitoring station 326. The computation of amine consumption in the second process 315 could be made from the initial amine feed rate determination and amine consumption in the first process 314 determination, or instead it could be made using the determinations made at the second sampling/monitoring station 325 whereat the both the actual inert tracer and amine concentrations were determined and hence are in a second known proportion. In other words, the measurements of the inert tracer concentration and the actual amine residual level at the second sampling/monitoring station 325 provide information equivalent to a feed ratio between tracer and amine and feed rate for the second process 315.

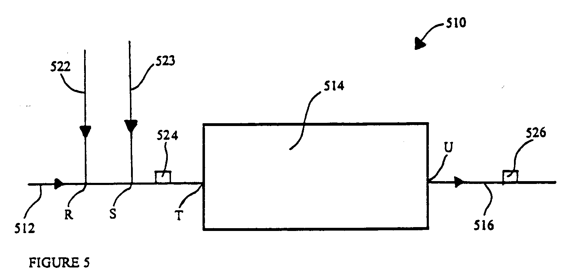

Referring now to FIGURE 5, there is shown schematically a process plant 510 including a raw material line 512 which feeds into a process 514 at point T, a product outlet line 516 from the process 514 (commencing at point U) which discharges the product. Along the raw material line 512 is a phosphate feed line 522 and an alum feed line 523, which introduce phosphate and alum to the raw material at respectively points R and S, both ahead of the process 514. The phosphate and alum are both consumed during the processing of the raw material in the process 514. The phosphate feed that is introduced to the raw material at point R contains a first inert tracer in known proportion to the phosphate. The alum feed that is introduced to the raw material at point S contains a second tracer in known proportion to the alum in the feed. Between points S and T is a first sampling/monitoring station 524. After point U is a second sampling/monitoring station 526. The first sampling/monitoring station 524 determines the concentration of both the first and second inert tracers and thus the separate phosphate and alum feed rates based on the concentrations of the inert tracers at that point and the known proportions between the level of such inert tracers and the level of the additives as these combinations are fed to the raw material line 512. The second sampling/monitoring station 526 measures the separate concentrations of the inert tracers and thus provides the separate hypothetical zero-consumption concentrations of the phosphate and the alum. The separate residual concentrations of the phosphate and the alum are also determined at the second sampling/monitoring station 526. The difference between the actual additive levels as measured at the second sampling/monitoring station 526 and such zero-consumptions additive level determinations provides the separate phosphate and alum consumption rates.

The present invention is a A method of monitoring the consumption rate of at least one additive to a industrial process in a industrial process wherein the additive is added to at least one upstream process stream that feeds into at least one process zone in which the additive is at least partially consumed, and any additive residual leaves the process zone in at least one downstream process stream, comprising:

adding the additive to the process stream or each of the upstream process streams in an additive feed that contains an inert tracer in known proportion to the additive;

monitoring the concentration of the inert tracer in the upstream process stream or each of the upstream process streams and in the downstream process stream or each the downstream process streams;

determining the concentration of the additive residual in the downstream process stream or in each of the downstream process streams;

calculating the rate of additive consumption in the process zone; and

optionally making a consumption-responsive additive feed rate adjustment.

In preferred embodiments, the inert tracer is a fluorescent specie and wherein the monitoring of the inert tracer is a fluorometric monitoring. The monitoring is a on a singular, intermittent, semi-continuous or continuous basis, and is conducted on-site.

The industrial process may have a plurality of process zones in which the additive is at least partially consumed and a plurality of process streams, wherein each of the process zones is serviced by both an upstream and downstream process stream, wherein the inert tracer concentration is monitored in a plurality of the upstream process streams and in a plurality of the downstream process streams, wherein the additive residual concentration is determined in a plurality of the downstream process streams, wherein a single process stream may be an upstream process stream for one of the plurality of process zones and a downstream process stream for another of the plurality of process zones, and wherein the rate of additive consumption is calculated for a plurality of the process zones.

The industrial process may have a plurality of process zones in which the additive is at least partially consumed, the process zones being disposed sequentially, and a plurality of process streams, wherein the sequence of process zones is serviced by both an upstream process stream, at least one intermediate process stream that is downstream of one of the process zones and upstream of one of the process zones, and downstream process stream, wherein the inert tracer concentration is monitored in a the upstream process stream, the intermediate process stream and in the downstream process stream, wherein the additive residual concentration is determined in the intermediate process stream and in the downstream process stream, and wherein the rate of additive consumption is calculated for a plurality of the process zones.

The industrial process may have a plurality of process zones in which the additive is at least partially consumed, the process zones being disposed in parallel, and a plurality of process streams, wherein the parallel process zones are each serviced by separate upstream process streams and separate downstream process streams, wherein the additive feed is added to a common upstream process stream that partially feeds into each of the separate upstream process streams; wherein the inert tracer concentration is monitored in the upstream process streams and the downstream process streams, wherein the additive residual concentration is determined in the downstream process streams, and wherein the rate of additive consumption is calculated for a plurality of the process zones.

In preferred embodiment, the additive feed contains a sufficient amount of the inert tracer to provide a concentration of the inert tracer in the downstream process stream of from about 0.5 ppb to about 100 ppm and more preferably from about 5 ppb to about 7 ppm.

The amount of the inert tracer added to the process stream and the wavelengths of excitation and emission selected for sensing the inert tracer's fluorescence emissivity preferably provide a relative fluorescence of the inert tracer of at least about 5 for the background fluorescent of the process stream. The inert tracer preferably is a naphthalene monosulfonic acid, a naphthalene disulfonic acid, a naphthalene trisulfonic acid, a sulfonated pyrene or a water soluble salt thereof, particularly 2-naphthalene sulfonic acid, 1,5-naphthalene disulfonic acid, or 1,3,6,8-pyrene.

Unless expressly indicated otherwise here, the inclusion of a prefix or suffix in parenthesis designates the word with such prefix or suffix as an alternative. For instance, "specie(s)" means "specie and/or species", "determination(s)" means "determination and/or determinations", "technique(s)" means "technique and/or techniques", "location(s)" means, "chemical(s)" means "chemical and/or chemicals", "component(s)" means "component and/or components", "tracer(s)" means "tracer and/or tracers", and the like. By "ppm" is meant "parts per million" by weight. By "ppb" us meant "parts per billion" by weight.

## Industrial Applicability of the Invention

The present invention is applicable to all industries employing an additive for a process in which it is consumed for which a consumption rate of such additive is desired and for which an inert tracer is available.

## Claims

1. A method of monitoring the consumption rate of at least one additive to an industrial process in an industrial process wherein said additive is added to at least one upstream process steam that feeds into at least one process zone in which said additive is at least partially consumed, and any additive residual leaves said process zone in at least one downstream process stream, comprising:

adding said additive to said process stream or each of said upstream process streams in an additive feed that contains an inert tracer in known proportion to said additive;

monitoring the concentration of said inert tracer in said upstream process stream or each of said upstream process streams and in said downstream process stream or each said downstream process streams;

determining the concentration of said additive residual in said downstream process steam or in each of said downstream process streams;

calculating the rate of additive consumption in said process zone; and

optionally making a consumption-responsive additive feed rate adjustment.

2. The method of Claim 1 wherein said inert tracer is a fluorescent specie and wherein said monitoring of said inert tracer is a fluorometric monitoring.

3. The method of Claim 1 or 2 wherein said monitoring is a on a singular, intermittent, semi-continuous or continuous basis, and is conducted on-site.

4. The method of any of claims 1-3 wherein said industrial process has a plurality of process zones in which said additive is at least partially consumed and a plurality of upstream and downstream process streams,

wherein each of said process zones is serviced by both an upstream and downstream process stream,

wherein said inert tracer concentration is monitored in a plurality of said upstream process streams and in a plurality of said downstream process streams,

wherein said additive residual concentration is determined in a plurality of said downstream process streams,

wherein a single process stream may be an upstream process stream for one of said plurality of process zones and a downstream process stream for another of said plurality of process zones, and

wherein said rate of additive consumption is calculated for a plurality of said process zones.

5. The method of any of Claims 1-3 wherein said industrial process has a plurality of process zones in which said additive is at least partially consumed, said process zones being disposed sequentially, and a plurality of process streams,

wherein said sequence of process zones is serviced by both an upstream process stream, at least one intermediate process stream that is downstream of one of said process zones and upstream of one of said process zones, and downstream process stream,

wherein said inert tracer concentration is monitored in a said upstream process stream, said intermediate process stream and in said downstream process stream,

wherein said additive residual concentration is determined in said intermediate process stream and in said downstream process stream, and

wherein said rate of additive consumption is calculated for a plurality of said process zones.

6. The method of any of Claims 1-3 wherein said industrial process has a plurality of process zones in which said additive is at least partially consumed, said process zones being disposed in parallel, and a plurality of process streams,

wherein said parallel process zones are each serviced by separate upstream process streams and separate downstream process streams,

wherein said additive feed is added to a common upstream process stream that partially feeds into each of said separate upstream process streams;

wherein said inert tracer concentration is monitored in said upstream process streams and said downstream process streams,

wherein said additive residual concentration is determined in said downstream process streams, and

wherein said rate of additive consumption is calculated for a plurality of said process zones.

7. The method of any of Claims 2-6 wherein said additive feed contains a sufficient amount of said inert tracer to provide a concentration of said inert tracer in said downstream process stream of from about 0.5 ppb to about 100 ppm.

8. The method of Claim 7 wherein said additive feed contains a sufficient amount of said inert tracer to provide a concentration of said inert tracer in said downstream process stream of from about 5 ppb to about 7 ppm.

9. The method of any of Claims 2-8 wherein the amount of said inert tracer added to said process stream and the wavelengths of excitation and emission selected for sensing said inert tracer's fluorescence emissivity provide a relative fluorescence of said inert tracer of at least about 5 for the background fluorescent of said process stream.

10. The method of any of Claims 2-9 wherein said inert tracer is a napahthalene monosulfonic acid, a naphthalene disulfonic acid, a naphthalene trisulfonic acid, a sulfonated pyrene or a water soluble salt thereof.

11. The method of Claim 10 wherein said inert tracer is 2-naphthalene sulfonic acid or a water soluble salt thereof.

12. The method of Claim 10 wherein said inert tracer is 1,5-naphthalene disulfonic acid or a water soluble salt thereof.

13. The method of Claim 10 wherein said inert tracer is 1,3,6,8-pyrene tetrasulfonic acid or a water soluble salt thereof.

14. A method of monitoring the consumption rate of at least one additive to an industrial process in an industrial process wherein said additive is added to a plurality of upstream process streams that all feed into a process zone, said additive is at least partially consumed within said process zone, and any additive residual leaves said process zone in a downstream process stream, comprising:

adding said additive to each of said upstream process streams in an additive feed that contains an inert tracer in known proportion to said additive;

monitoring the concentration of said inert tracer in each of said upstream process streams and in said downstream process stream;

determining the concentration of said additive residual in said downstream process stream;

calculating the rate of additive consumption; and

optionally making a consumption-responsive additive feed rate adjustment.

15. The method of Claim 14 wherein said inert tracer is a fluorescent specie and wherein said monitoring of said inert tracer is a fluorometric monitoring.

16. The method of Claim 15 wherein additive feed contains a sufficient amount of said inert tracer to provide a concentration of said inert tracer in said downstream process stream of from about 0.5 ppb to about 100 ppm.

17. A method of monitoring the consumption rate of at least one additive to an industrial process in an industrial process wherein said additive is added to an upstream process stream that feeds into a process zone, said additive is at least partially consumed within said process zone, and any additive residual leaves said process zone in a plurality of downstream process streams, comprising:

adding said additive to said upstream process stream in an additive feed that contains an inert tracer in known proportion to said additive;

monitoring the concentration of said inert tracer in said upstream process stream and in each of said downstream process streams;

determining the concentration of said additive residual in each of said downstream process streams;

calculating the rate of additive consumption; and

optionally making a consumption-responsive additive feed rate adjustment.

18. The method of Claim 17 wherein said inert tracer is a fluorescent specie and wherein said monitoring of said inert tracer is a fluorometric monitoring.

19. The method of Claim 18 wherein said additive feed contains a sufficient amount of said inert tracer to provide a concentration of said inert tracer in said downstream process stream of from about 0.5 ppb to about 100 ppm.

20. The method of Claim 19 wherein said inert tracer is 2-naphthalene sulfonic acid, 1,5-naphthalene disulfonic acid, 1,3,6,8-pyrene tetrasulfonic acid or water soluble salts thereof.

FIGURE 1

FIGURE 2

FIGURE 3

FIGURE 4

FIGURE 5

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| D,X<br>Y<br>A | US-A-4 783 314 (HOOTS J E ET AL)<br><br><br>* column 2, line 16 - column 3, line 27; column 5, lines 46-62; column 9, lines 1-21 *<br>--- | 1-11,<br>14-20<br>12<br>13 | G01N33/18 |
| X<br><br>A | EP-A-0 504 520 (NALCO CHEMICAL COMPANY)<br><br><br><br>* page 2, line 55 - page 3, line 30;page 7, lines 9-41; claim 3; figure 7 *<br>--- | 1-3,<br>9-11,<br>17-20<br>4-8,<br>14-16 | |
| Y<br>A | EP-A-0 475 602 (NALCO CHEMICAL COMPANY)<br><br><br>* example 37; table VII *<br>--- | 12<br>1,4,<br>7-10,20 | |
| A | WO-A-90 05916 (DOWBEN R M)<br>* page 9, line 1 - page 11, line 10; page 12, lines 8-21; page 17, lines 12-20 *<br>--- | 10,13,20 | **TECHNICAL FIELDS SEARCHED** (Int.Cl.5)<br><br>G01N |
| A | US-A-5 132 432 (HAUGLAND R P ET AL)<br>* column 6, line 7 - column 7, line 8; column 8, lines 45-64; tables 2,4 *<br>--- | 10,13,20 | |
| P,A | US-A-5 246 860 (HUTCHINS R D ET AL)<br><br><br>* column 1, line 6 - column 2, line 30; column 5, line 14 - column 9, line 35; tables I,IV *<br>----- | 1-7,<br>10-12,<br>14-20 | |

The present search report has been drawn up for all claims

| Place of search | Date of completion of the search | Examiner |
|---|---|---|
| BERLIN | 25 August 1994 | Johnson, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document